# FASCICULE DE BREVET EUROPEEN

(11) **EP 0 730 859 B1**
(45) Date de publication et mention de la délivrance du brevet: **24.06.1998**
(21) Numéro de dépôt: 96400239.8
(22) Date de dépôt: 05.02.1996
(51) Int. Cl.: A61K 7/48, A61K 7/027

(54) **Composition cosméthique sous forme de dispersion solide comprenant une phase grasse, un alcool polyhydrique et des charges**
Kosmetisches Mittel in Form einer festen Dispersion bestehend aus einer Fettphase, einem mehrwertigen Alkohol und Zusatzstoffen
Cosmetic composition in form of a solid dispersion containing a fatty phase, a polyhydric alcohol and charges

(30) Priorité: 03.03.1995 FR 9502522
(43) Date de publication de la demande: 11.09.1996
(73) Titulaire: L'OREAL, 75008 Paris (FR)
(72) Inventeur: Miguel-Colombel, Dolorès, F-92340 Bourg-la-Reine (FR)
(74) Mandataire: Dodin, Catherine

(56) Documents cités:
- EP-A- 0 524 892
- EP-A- 0 609 132
- WO-A-94/06400
- WO-A-94/06401

## Description

La présente invention a trait à une composition cosmétique susceptible d'être appliquée sur la peau, notamment sur les lèvres en tant que produit de soin et/ou de maquillage.

Il est connu, par exemple par le document FR2679467 une composition cosmétique telle qu'un rouge à lèvres, un fard à joues ou un fond de teint, se présentant sous forme d'une dispersion solide anhydre comprenant principalement un alcool polyhydrique, au moins un constituant gras et des pigments minéraux et/ou organiques usuels.
Par dispersion solide, on entend une composition solide entre 0-50°C, ce qui correspond au domaine de température de conservation et d'utilisation des produits cosmétiques. Ces dispersions solides peuvent se présenter sous forme de produits gras coulés et notamment sous forme de bâton, pour le rouge à lèvres en particulier.
Or, on a constaté avec étonnement que lorsque l'on prépare une composition cosmétique similaire à celle décrite dans cet art antérieur, mais exempte de pigment c'est-à-dire de charge colorée usuellement employée comme agent colorant en maquillage, la composition obtenue ne possède plus la consistance adéquate; sa consistance devient en effet très molle, à tel point que la composition, par exemple se présentant sous forme de bâton de rouge à lèvres, peut "s'écraser" sur la peau lorsqu'on l'applique. On dépose alors sur les lèvres une quantité excessive de produit, de manière non homogène.

La présente invention a pour but de pallier cet inconvénient et de proposer une dispersion anhydre solide exempte de pigments, tout en possédant une consistance adéquate qui permet son application de manière usuelle.

La présente invention a donc pour objet l'utilisation d'au moins 5% en poids de charges non colorées, dans une composition cosmétique anhydre, exempte de pigments, comprenant une dispersion solide d'au moins une phase grasse et d'au moins un alcool polyhydrique, pour obtenir une composition sous forme de bâton, possédant une consistance adéquate qui permet son application de manière usuelle.
Ces charges peuvent être choisies parmi l'amidon, éventuellement traité par l'anhydride octénylsuccinique, les microsphères de silice, les microbilles de résine silicone et/ou la poudre de Nylon

Les compositions obtenues présentent de bonnes propriétés émollientes par application sur la peau, ainsi qu'une bonne tenue.

Par pigment, on entend dans la présente description les composés minéraux colorés généralement utilisés pour donner de la couleur aux produits de maquillage, parmi lesquels on peut citer les oxydes de fer ou de chrome.

Parmi les charges non colorées, donc généralement blanches, susceptibles d'être incorporées dans la composition selon l'invention, on peut citer des charges minérales telles que l'amidon, éventuellement traité par l'anhydride octénylsuccinique, les microsphères de silice ou les microbilles de résine silicone, et/ou des charges organiques telles que la poudre de Nylon. Ces charges sont présentes à raison d'au moins 5% en poids par rapport au poids total de la composition, de préférence à raison de 7-15%. Elles peuvent toutefois être présentes en quantité importante, par exemple de l'ordre de 28-30% en poids.

Selon l'invention, la phase grasse peut comprendre des huiles et/ou des cires. Le point de fusion finissant de ladite phase grasse est de préférence inférieur à 110°C, ce qui n'empêche pas que certains constituants puissent avoir un point de fusion supérieur. Il est préférable d'utiliser au moins une cire de point de fusion supérieur à 60°C.

Parmi les huiles, on peut citer :
. les huiles minérales telles que l'huile de paraffine ou de vaseline,
. les huiles animales telles que le perhydrosqualène ou l'huile d'arara,
. les huiles végétales telles que l'huile d'amande douce, de calophyllum, de palme, de ricin, d'avocat, de jojoba, d'olive ou de germes de céréales,
. les huiles de silicone telles que le diméthylpolysiloxane,
. des esters d'acide lanolique, d'acide oléique, d'acide laurique, d'acide stéarique ou d'acide myristique par exemple,
. des alcools tels que l'alcool oléique, l'alcool linoléique ou linolénique, l'alcool isostéarique ou l'octyl dodécanol,
. des acétylglycérides, des octanoates, décanoates ou ricinoléates d'alcools ou de polyalcools.

Parmi les cires, on peut citer :
. les cires minérales telles que les cires microcristallines, la paraffine, le pétrolatum, la vaseline, l'ozokérite, la cire de montan,
. les cires animales telles que la cire d'abeilles, la lanoline et ses dérivés,
. les cires végétales telles que la cire de Candellila, la cire de Carnauba, la cire du Japon, le beurre de cacao,
. les huiles hydrogénés, les esters gras et les glycérides concrets à 25°C,
. l'alcool cétylique, l'alcool stéarylique, la colophane et ses dérivés.
La phase grasse peut représenter 20-94% en poids de la composition, de préférence 40-85%.

Selon l'invention, l'alcool polyhydrique peut être un composé ayant 2-8 atomes de carbone et 2-6 fonctions hydroxyles, tel que l'éthylène glycol, le glycérol, le propane-1,2 diol, la diglycérine, l'érythritol, l'arabitol, l'adonitol, le sorbitol et le dulcitol. L'alcool polyhydrique peut également être un polyéther alcool de poids moléculaire moyen de 150-600, tel que le polyéthylène glycol 300 et la polyglycérine 500. L'alcool polyhydrique peut représenter 0,5-50% en poids de la composition, de préférence 5-30%.

On peut encore ajouter tout additif usuellement utilisé en cosmétique, tel que des antioxydants, des parfums, des conservateurs, des actifs cosmétiques, des vitamines, des acides gras essentiels, des sphingolipides, des filtres solaires liposolubles, des tensioactifs, des polymères liposolubles comme les polyalkylènes, les polyacrylates et les polymères siliconés compatibles avec les corps gras. L'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions comportant au moins 5% en poids de charges non colorées peuvent être préparées de manière usuelle selon l'état de la technique, par l'homme du métier sur base de ses connaissances générales. Les compositions se présenter sous forme d'un produit de maquillage et/ou de soin, et en particulier sous la forme d'une base de soin pour les lèvres.

L'invention est illustrée plus en détail dans les exemples suivants.

### Exemple 1 - exemple selon l'invention

On prépare une émulsion solide sous forme d'un stick pour les lèvres ayant la composition suivante (% en poids) :

| | |
|---|---|
| . huile de ricin | 15% |
| . huile de jojoba | 15% |
| . huile de coco hydrogénée | 7,8% |
| . tri-stéarate de glycéryle | 3% |
| . lanolate d'isopropyle | 30% |
| . cire de Carnauba | 3% |
| . cire de polyéthylène | 10% |
| . glycérine | 8% |
| . microbiles de résine silicone | 8% |
| . antioxydant | 0,2% |

Le bâton est préparé de la façon suivante : on chauffe d'une part la phase grasse, d'autre part l'alcool polyhydrique, à une température d'environ 100°C, on ajoute la charge puis l'on mélange le tout à l'aide d'une turbine Moritz à la vitesse de 3000 tr/min. On peut alors couler ledit mélange dans des moules adéquats. La composition obtenue est anhydre et incolore.
Après 24 heures dans une étuve régulée à 34°C, la composition se présente toujours sous forme d'un bâton de consistance normale, et s'applique de manière adéquate. La base traitante ainsi préparée est aisée et agréable à appliquer (douce et glissante).

### Exemple 2 - exemple comparatif

On prépare une émulsion solide sous forme d'un stick pour lèvres ayant la composition suivante (% en poids) :

| | |
|---|---|
| . huile de ricin | 17% |
| . huile de jojoba | 17% |
| . huile de coco hydrogénée | 7,8% |
| . tri-stéarate de glycéryle | 3% |
| . lanolate d'isopropyle | 34% |
| . cire de Carnauba | 3% |
| . cire de polyéthylène | 10% |
| . glycérine | 8% |
| . antioxydant | 0,2% |

### Le stick est préparé selon l'exemple 1.

Après 24 heures dans une étuve à 34°C, la composition s'écrase complètement lors de son application sur les lèvres, et l'on constate que la moitié, voire la totalité du stick se retrouve sur les lèvres.

## Revendications

1. Utilisation d'au moins 5% en poids de charges non colorées, dans une composition cosmétique anhydre, exempte de pigments, comprenant une dispersion solide d'au moins une phase grasse et d'au moins un alcool polyhydrique, pour obtenir une composition sous forme de bâton, possédant une consistance adéquate qui permet son application de manière usuelle.

2. Utilisation selon la revendication 1, dans laquelle les charges non colorées sont choisies parmi l'amidon, éventuellement traité par l'anhydride octénylsuccinique, les microsphères de silice, les microbilles de résine silicone et/ou la poudre de Nylon.

3. Utilisation selon l'une des revendications précédentes, dans laquelle les charges non colorées sont présentes à raison de 5-30% en poids, de préférence 7-15% en poids, dans la composition.

4. Utilisation selon l'une des revendications précédentes, dans laquelle la phase grasse comprend au moins une cire de point de fusion supérieur à 60°C.

5. Utilisation selon l'une des revendications précédentes, dans laquelle l'alcool polyhydrique est choisi parmi les composés ayant 2-8 atomes de carbone et 2-6 fonctions hydroxyles, tel que l'éthylène glycol, le glycérol, le propane-1,2 diol, la diglycérine, l'érythritol, l'arabitol, l'adonitol, le sorbitol et le dulcitol, et/ou parmi les polyéthers alcool de poids moléculaire moyen de 150-600, tel que le polyéthylène glycol 300 et la polyglycérine 500.

6. Utilisation selon l'une des revendications précédentes, dans laquelle l'alcool polyhydrique est présent à raison de 0,5-50% en poids dans la composition, de préférence 5-30% en poids.

7. Utilisation selon l'une des revendications précédentes, dans une composition de base de soin pour les lèvres.

## Claims

1. Use of at least 5% by weight of colourless fillers in an anhydrous, pigment-free cosmetic composition comprising a solid dispersion of at least one fatty phase and of at least one polyhydric alcohol, in order to obtain a composition in stick form, having an adequate consistency which allows it to be applied in the usual way.

2. Use according to Claim 1, in which the colourless fillers are chosen from starch, optionally treated with octenylsuccinic anhydride, silica microspheres, silicone resin ballotini and/or nylon powder.

3. Use according to either of the preceding claims, in which the colourless fillers are present in a proportion of 5-30% by weight, preferably 7-15% by weight, in the composition.

4. Use according to one of the preceding claims, in which the fatty phase comprises at least one wax with a melting point above 60°C.

5. Use according to one of the preceding claims, in which the polyhydric alcohol is chosen from compounds having 2-8 carbon atoms and 2-6 hydroxyl functions, such as ethylene glycol, glycerol, 1,2-propanediol, diglycerol, erythritol, arabitol, adonitol, sorbitol and dulcitol, and/or from polyether alcohols with an average molecular weight of 150-600, such as polyethylene glycol 300 and polyglycerol 500.

6. Use according to one of the preceding claims, in which the polyhydric alcohol is present in a proportion of 0.5-50% by weight in the composition, preferably 5-30% by weight.

7. Use according to one of the preceding claims, in a care base compositon for the lips.

## Patentansprüche

1. Verwendung von mindestens 5 Gew.-% farbloser Füllstoffe in einer wasserfreien kosmetischen Zusammensetzung, die keine Pigmente enthält und eine feste Dispersion mindestens einer Fettphase und mindestens eines mehrwertigen Alkohols umfaßt, zur Herstellung einer Zusammensetzung in Form eines Stiftes, die eine geeignete Konsistenz aufweist, die es ermöglicht, die Zusammensetzung in üblicher Weise aufzutragen.

2. Verwendung nach Anspruch 1, worin die farblosen Füllstoffe unter Stärke, die gegebenenfalls mit Octenylbernsteinsäureanhydrid behandelt ist, Mikrokugeln aus Siliciumdioxid, Mikrokügelchen aus Siliconharz und/oder Nylonpulver ausgewählt sind.

3. Verwendung nach einem der vorhergehenden Ansprüche, worin die farblosen Füllstoffe in der Zusammensetzung in einem Mengenanteil von 5 bis 30 Gew.-%, vorzugsweise von 7 bis 15 Gew.-%, vorliegen.

4. Verwendung nach einem der vorhergehenden Ansprüche, worin die Fettphase mindestens ein Wachs mit einem Schmelzpunkt von über 60 °C umfaßt.

5. Verwendung nach einem der vorhergehenden Ansprüche, worin der mehrwertige Alkohol ausgewählt ist unter den Verbindungen mit 2 bis 8 Kohlenstoffatomen und 2 bis 6 Hydroxygruppen, wie Ethylenglykol, Glycerin, 1,2-Propandiol, Diglycerin, Erythrit, Arabit, Ribit, Sorbit und Dulcit, und/oder unter den Polyetheralkoholen mit einem mittleren Molekulargewicht von 150 bis 600, wie Polyethylenglykol 300 und Polyglycerin 500.

6. Verwendung nach einem der vorhergehenden Ansprüche, worin der mehrwertige Alkohol in der Zusammensetzung in einem Mengenanteil von 0,5 bis 50 Gew.-%, vorzugsweise von 5 bis 30 Gew.-%, vorliegt.

7. Verwendung nach einem der vorhergehenden Ansprüche in einer Zusammensetzung einer Pflegegrundmasse für die Lippen.
